(19) Europäisches Patentamt
European Patent Office
Office européen des brevets

(11) **EP 1 643 935 B1**

(12) **EUROPEAN PATENT SPECIFICATION**

(45) Date of publication and mention
of the grant of the patent:
**26.08.2009 Bulletin 2009/35**

(51) Int Cl.:
***A61F 2/04*** *(2006.01)*    ***A61L 27/58*** *(2006.01)*

(21) Application number: **04736291.8**

(22) Date of filing: **07.06.2004**

(86) International application number:
**PCT/SE2004/000879**

(87) International publication number:
**WO 2005/002472 (13.01.2005 Gazette 2005/02)**

(54) **A DEVICE AND KIT FOR PROMOTING REGENERATION OF AN INJURED NERVE**

VORRICHTUNG UND BAUSATZ ZUR FÖRDERUNG DER REGENERATION EINES VERLETZTEN NERVS

DISPOSITIF ET KIT DESTINE A FAVORISER LA REGENERATION D'UN NERF LESE

(84) Designated Contracting States:
**AT BE BG CH CY CZ DE DK EE ES FI FR GB GR HU IE IT LI LU MC NL PL PT RO SE SI SK TR**

(30) Priority: **03.07.2003 SE 0301974**

(43) Date of publication of application:
**12.04.2006 Bulletin 2006/15**

(73) Proprietor: **Neurogen Medical Innovation i Umeå AB**
**870 33 Docksta (SE)**

(72) Inventors:
• **OLAUSSON, Susanne**
**S-443 38 Lerum (SE)**
• **WIBERG, Mikael**
**S-870 33 Docksta (SE)**
• **RINDLAV WESTLING, Asa**
**S-437 32 Lindome (SE)**

(74) Representative: **Andersson, Inga-Lill et al**
**Awapatent AB**
**Södra Hamngatan 37-41**
**Box 11394**
**404 28 Göteborg (SE)**

(56) References cited:
EP-A1- 0 945 145    EP-A1- 1 254 671
US-A- 5 358 475    US-A- 5 599 552
US-A- 5 735 863    US-B1- 6 548 569

## Description

Technical field

The present invention relates to the field of nerve regeneration. More specifically, the invention relates to a device for promoting regeneration of an injured nerve, a kit for preparing such a device, and a biodegradable sheet for preparing such a device. The invention also relates to biodegradable guiding means for promoting regeneration of an injured nerve.

Technical background

The anatomic nervous system consists of the central nervous system (CNS) and the peripheral nervous system (PNS), and comprises nerve cells, which are supported and protected by glial cells, such as Schwann cells.

A nerve cell (neuron) comprises a nerve cell body, from which dendrites and an axon extend. Axons are termed nerve fibres, and a nerve is a bundle of several nerve fibers.

Chemical stimulation of a neuron generates a nerve impulse that can pass between two or several neurons and the junction between two separate neurons is called a synapse.

The CNS consists of the brain and the spinal cord. Nerve cell bodies in the CNS are known as grey matter. So-called white matter in the CNS consists primarily of axons coated with a light-coloured supporting and insulating myelin sheet produced by glial cells.

Spinal nerves extend from the spinal cord and cranial nerves extend from the brainstem. The axons of these nerves transmit signals between the CNS and the rest of the body, and constitute the PNS. A cluster of nerve cell bodies on a peripheral nerve is called a ganglion. Thus, nerve cell bodies are located in the brain, in the spinal cord, and in peripheral ganglia.

The axons in the PNS is either non-myelinated or myelinated. The nerve signal transmission is faster in myelinated nerve fibres. The insulating and supporting myelin sheet surrounding peripheral nerve fibres is produced by so-called Schwann cells.

Schwann cells also produce neurotrophic factors (nerve growth promoting substances) essential for nerve cell growth and function.

When a nerve is injured, a gap is formed whereby proximal and distal nerve portions are located at the gap. To bridge the gap and substantially re-establish nerve function, axons in the proximal end of the injured nerve must navigate to reach the corresponding distal end of the injured nerve. Injury to an axon stimulates production of neurotrophic factors, which promote growth of the proximal nerve end. The proximal end of the injured nerve also senses signals from other cells in the surroundings and determine the rate and direction of nerve growth.

After a lag period of approximately two weeks, during which the nerve growth is paralysed, the repair processes of the injured nerve start. Each myelinated axon of the injured nerve divides into a multiplicity of fine regenerating axon sprouts with finger-like extensions which grow out from the proximal nerve end. The growth rate is generally 1-2 mm/day. When these sprouts reaches the distal end and contact has been established between the two nerve ends, the Schwann cells are stimulated to proliferate and form a basal lamina of collagen, proteoglycans, and laminin. Moreover, regeneration of a larger number of axons is initiated, i.e. the quantity of axons is increased.

The sprouts extending from the proximal nerve end typically grow in many directions and unless the gap is small, the axon sprouts from the proximal end may never establish contact with the corresponding distal end, thus resulting in permanent loss of nerve function.

Moreover, soft tissue damage, scar tissue formation and disruption of blood supply may interrupt the natural nerve regeneration process.

Nerve defects have traditionally been reconstructed either by directly suturing together the ends of the split nerve or by surgical transfer of a part of a healthy nerve from an uninjured location to the injured site. The healthy nerve is in the vast majority of cases taken from the patient (autograft) and only rarely is the nerve graft taken from a donor (allograft). These procedures are all difficult, expensive and not always successful.

Another approach for nerve regeneration is the use of a nerve conduit (also referred to as nerve tube, nerve channel, nerve guide, tubular nerve prosthesis, etc).

The material constituting the nerve conduit should preferably be biocompatible, biodegradable, non-toxic, non-carcinogenic, non-antigenic and should exhibit desirable mechanical properties, such as strength, flexibility, elasticity, and processibility. Furthermore, the material should preferably be porous to allow passage of substances essential for nerve cell metabolism, such as water, salts, nutrients, etc. It may be noted that these considerations also apply to the invention.

Both inert biocompatible tubes, such as conduits of silicone, polyethylene, poly(vinyl chloride), poly(tetra-fluorethylene), and biocompatible biodegradable conduits, such as conduits made of poly(glycolic acid) (PGA), poly(lactic acid) (PLA), poly(lactic-co-glycolic acid) (PLGA), hyaluronic acid, collagen, gelatine, or biological tissue, have been suggested for this purpose.

Preferably, the conduit is arranged such as the ends of the injured nerve will be located inside the lumen of the nerve conduit and by leaving a small gap between the nerve ends, the conduit optimises the growth of the axon sprouts from the proximal nerve end in the proper direction towards the distal nerve end lead by endogeneous neurotrophic guidance. Furthermore, the nerve conduit provides support to the fragile growing nerve and increases the local concentration of endogenous neurotrophic factors released around the growing nerve.

The use of a nerve conduit generally results in an increase in the number and/or size of regenerated axons and a decrease in the time required for regeneration as compared to natural nerve regeneration without the use of a nerve conduit.

To further promote nerve regeneration, it has been suggested, for instance in WO 97/37002, to add nerve growth promoting substances, such as nerve growth factor (NGF); brain-derived neurotrophic factor (BDNF); neurotrophin-3 (NT-3; neurotrophin-4 (NT-4); glial growth factor (GGF); insulin-like growth factor (IGF), including a variant of IGF called mechano-growth factor (MGF); platelet-derived growth factor (PDGF); fibroblast growth factor (FGF); transforming growth factor (TGF); epidermal growth factor (EGF); fibronectin; fibrin; laminin; cells, such as Schwann cells, stem cells and precursor cells thereof, endothelial cells, and fibroblasts; nutrients; nerve tissue extract, and/or other biologically active substances or cells, at the place of injury , such as within the lumen of the conduit.

Matrix materials, such as hydrogels, e.g. poly(ethylene oxide), hyaluronate, collagen, agarose, chitosan, methylcellulose, or alginate, comprised within the lumen of the nerve conduit have also been suggested, for instance in WO 97/37002, to promote the nerve regeneration, especially for regeneration of larger nerve gaps within the range of from 1 cm to 10 cm. It has also been suggested to disperse nerve growth promoting substances or cells in the matrix material.

Mechanical nerve guide structures (orientation aids) on the interior wall surface of the nerve conduit or in the lumen of the nerve conduit have also been suggested.

WO 01/81552 describes a patterned interior wall surface having nerve guiding grooves.

WO 88/06871 describes a nerve conduit having a plurality of guide channels in the lumen. The channels may, for instance, be defined between and/or through a plurality of longitudinally extending fibres, i.e. solid compact fibres or hollow fibres.

Also WO 97/37002 discloses mechanical guide means for guided tissue regeneration, e.g. in the form of fibres.

EP 1 201 256 describes a microporous guide tube of polymers of hydroxycarboxylic acids, optionally having several monofilaments of polymers of hydroxycarboxylic acids located in the guide tube, wherein the resorbability of the guide tube and the fibres decrease over their lengths. It is stated that the regenerated nerve should be exposed as early as possible so as to permit a normal metabolism with the environment. The guide tube and the fibres are thus more rapidly degraded at the proximal nerve end than at the distal nerve end. This is, for instance, obtained via the use of polymers having different molecular weights. The in vivo degradation time is stated to be from 0.5 to 6 months along the length of the tube and fibres. The state of the art according to said document is acknowledged in the preambles of the independent claims.

EP 0945145 describes a nerve tube of a biodegradable mesh material, such as PGA, PLA or PLGA, with a coating of e.g. cross-linked type I collagen on the inner and outer surfaces of the tube. The tube comprises a collagen body of cross-linked type I collagen fibres forming longitudinal cavities filled with a matrix gel containing collagen, laminin, heparan sulfate proteoglycans, entactin and growth factor. It is stated that the collagen-coated tube should remain in the body until the nerve has finished regenerating. Since the fibres also are made of cross-linked collagen, the fibres will also remain in the body until the nerve has finished regenerating, that is until the completion of the entire nerve regeneration process.

US 6548569 describes compositions and device, such as nerve tubes, made of polyhydroxyalkanoates, e.g. P-4-HB.

Nevertheless, even though the time period required for establishing contact between the nerve ends is decreased using a nerve conduit having guiding means, such as fibres, within the lumen, it takes rather long time until the entire nerve has been regenerated to a satisfactory degree or, in some cases, a satisfactory nerve function is never attained.

Summary of the invention

The inventors have found that the use of guiding means, such as fibres, within the lumen of the nerve tube generally increases the rate of nerve regeneration, but often provide a rather low quantity of regenerated axons, i.e. a quantity insufficient to establish a satisfactory nerve function. Thus, the inventors have found that it would be a great advantage to obtain a higher quantity of regenerated axons, while maintaining a relatively high rate of nerve regeneration.

An object of the present invention is to alleviate the above problems and to improve the regeneration of injured nerves, in particular to increase the quantity of regenerated axons and thereby enhance the chance of obtaining a satisfactory degree of retrieved nerve function. More specifically, an object of the invention is to increase the rate of axon density growth.

According to a first aspect of the invention, this object is achieved with a device for promoting regeneration of an injured nerve according to claim 1.

Without being bound by any theory, it is believed that the guiding means, such as a plurality of fibres, initially should act as orientation aids for the axon sprouts until the regeneration process has established contact between the ends of the injured nerve (referred to as regenerated contact), i.e. until the axon sprouts extending from the proximal nerve end have established contact with the distal nerve end. Thereafter, the regenerated axons formed from the sprouts act as natural orientation aids for the succeeding growing axons and the guiding means are no longer required. It is believed that guiding means, such as fibres, may restrict or even block the growth of the succeeding growing axons and impair the amount of regenerated axons. Thus, according to the invention, a majority of the nerve guiding means, such as guiding fibres, should preferably be essentially disintegrated, more preferably completely degraded, when the regeneration process has established contact between the ends of the injured nerve.

When a nerve is injured, the nerve is generally paralysed for a period of about two weeks, said period being independent of the size of the gap. Thereafter the axons sprouts start to grow. The axon growth rate is generally about 1 mm/day, but the rate may vary within the range of from about 0.5 to about 2 mm/day.

Thus, a nerve gap of 1 cm is normally, when using a nerve conduit, bridged in about 19-34 days from the date of injury. The nerve then grows thicker by regeneration of more axons and myelination which takes about the same time as the bridging process, i.e. about 5-20 days for a 1 cm gap. Thus, the overall nerve regeneration process for a 1 cm gap would normally take approximately 1-2 months from the date of injury.

According to the invention, a majority of the guiding means, such as fibres, should preferably be essentially disintegrated, more preferably completely degraded, when the nerve gap has been bridged, i.e. when the regeneration process has established contact between the ends of the injured nerve. The nerve encasement structure should advantageously support the fragile growing nerve even after contact has been established between the nerve ends and said supporting structure should preferably last at least until the entire nerve regeneration process has been completed. The use of a porous nerve encasement structure has not been shown to negatively affect the regenerated nerve, so the structure may still be present a while after the nerve has been essentially regenerated and a satisfactory nerve function has been attained.

According to a second aspect of the invention, a kit for preparing a device for promoting regeneration of an injured nerve is provided in accordance with independent claim 13.

Other features and advantages of the present invention will become apparent from the following description of the invention.

Brief description of drawings

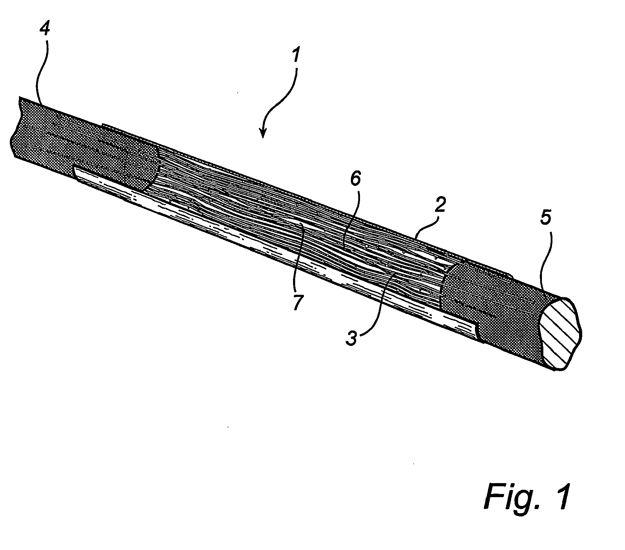

Fig. 1 schematically shows an embodiment of the device according to the invention.

Detailed description of the invention

As used herein the term "degradation" of a material means cleavage of molecular chains, such as polymer chains, constituting the molecule, thus reducing the molecular weight of the molecule. Degradation finally results in essentially complete mass loss. When the molecular weight of the molecule, e.g. a polymer, reaches the threshold level of water solubility of the breakdown products, rapid mass loss is observed, and the material is referred to as completely degraded.

As used herein the term "biodegradation" means degradation of a material (referred to as a biodegradable material) over time by enzymatic action, by hydrolytic (non-enzymatic) action, and/or by other similar mechanisms in the human body. As used herein "biodegradation" also includes dissolution of a material in body fluids without any molecular chain cleavage or molecular mass decrease and subsequent removal of the dissolved material by cellular activity. Thus, as used herein biodegradable materials also include materials that are generally referred to as bioabsorbable materials.

It shall be noted that as used herein, biodegradable materials include both completely and partly biodegradable (including bioabsorbable) materials. However, it shall be noted that when referring to the invention, the biodegradable material is preferably a completely biodegradable material.

As used herein the term "in vivo degradation time" means the time period from implantation of a biodegradable material in a mammal, including a human, and degradation until the molecular weight of the material has been reduced to such a level that an essentially disintegrated material has been attained, or alternatively, dissolution of a bioabsorbable material until an essentially disintegrated material has been attained.

As used herein, an essentially disintegrated material means a material that provides no substantial axon growth guiding function and no substantial axon growth blocking effect in vivo.

As used herein the term "biocompatible" means that a material, when implanted in a host, does not provoke a foreign body reaction from the host.

As used herein the term "bioresorbable" means biodegradation of a material and subsequent elimination of the degradation products through natural pathways.

As used herein the term "fibre" means a cylindrical or tubular structure wherein the length is much larger than its cross-sectional dimension. The fibres can either be solid or hollow.

As used herein the term "non-woven" means a type of fabric made directly from fibres or from a web of fibres without the preliminary yarn preparation needed for weaving or knitting. Non-woven may be compressed or non-compressed. A non-compressed non-woven may also be referred to as a non-bonded fibre web.

As used herein the term "porous" means a sufficiently open structure that allows for passage of physiological fluids, substances, and/or cells.

As used herein "one day" means 24 hours.

A first device not in accordance with the invention for promoting regeneration of an injured nerve comprises a nerve encasement structure and a plurality of biodegradable guiding means; preferably a plurality of biodegradable guiding fibres, wherein least a majority of the guiding means presents an in vivo degradation time $t_1$ being less than or approximately equal to a time $t_c$ required for establishing regenerated contact between the ends of an injured nerve using the device for said regeneration.

This means that the in vivo degradation time $t_1$ is selected such as when the guiding means, preferably in the form of fibres, by degradation (and/or dissolution) becomes essentially disintegrated, they will provide no substantial axon growth guiding function and no substantial axon growth blocking effect.

Said first device may alternatively be described as a device for promoting a process of regeneration of an injured nerve, said process presenting a pre-contact period extending from application of the device at the injured nerve up to a first occurrence of a re-established (regenerated) contact between the ends of the injured nerve, and post-contact period extending from the end of the pre-contact period and up to the end of the regeneration process, wherein said device comprises a nerve encasement structure and a plurality of guiding means, preferably fibres, which present an in vivo biodegradability being such that at least a majority of said guiding means becomes essentially disintegrated by degradation (and/or dissolution) during the pre-contact period.

It shall be noted that the in vivo degradation time $t_1$ generally is a distribution of a plurality of in vivo degradation times, such as $t_{1a}$ $t_{1b}$, $t_{1c}$, $t_{1d}$, etc, all of which fulfil the requirements of $t_1$.

Also the in vivo degradation time $t_2$ is generally a distribution of a plurality of in vivo degradation times, such as $t_{2a}$ $t_{2b}$, $t_{2c}$, $t_{2d}$, etc, all of which fulfil the requirements of $t_2$.

It shall also be noted that the term "nerve encasement structure" means a structure that at least partly encases the ends of the injured nerve.

The device according to the invention for promoting regeneration of an injured nerve may also be referred to as a nerve regeneration device.

The term "approximately" is in this context defined as about ± 20%, preferably about ± 10%.

Furthermore, the nerve encasement structure is preferably in the form of a tubular structure, such as a conduit.

Preferably, substantially all of the guiding means present an in vivo degradation time $t_1$ being less than or approximately equal to the time $t_c$ required for establishing contact between the ends of an injured nerve using the device for said regeneration.

The in vivo degradation time $t_1$ is preferably less than the time $t_c$ required for establishing regenerated contact between the ends of an injured nerve using the device for said regeneration.

Fig 1 shows a sectional schematic drawing of an embodiment of the device 1 according to the invention. The device 1 comprises a biodegradable nerve encasement structure 2 having a plurality of biodegradable guiding means in the form of fibres 3 within its lumen. The nerve encasement structure 2 encases the proximal and distal ends 4 and 5, respectively, of the injured nerve. The lumen of the nerve encasement structure 2 may comprise a hydrogel 6 and/or nerve growth promoting substances and/or cells, such as Schwann cells, 7 (described in more detail in the following text).

Preferably, at least a major part of the nerve encasement structure present an in vivo degradation time ($t_2$) being longer than the in vivo degradation time of the majority of the guiding means ($t_1$). Thus, the majority of the guiding means are in vivo preferably degraded faster than the major part of the nerve encasement structure.

In other words, at least a major part of the nerve encasement structure will not become disintegrated during the pre-contact period. Said major part of the nerve encasement structure becomes disintegrated during the post-contact period or afterwards.

Without being bound by any theory, the approximate time ($t_c$) required for establishing contact between the ends of an injured nerve using the device according to the invention may be expressed by Formula I:

$$\frac{L}{v} \leq t_c \leq 14 + \left(\frac{L}{v}\right) \qquad (\mathrm{I})$$

wherein
L = gap size [mm]
v = axon growth rate [mm/day]

As stated above, the axon growth rate is generally about 1 mm/day, but the rate may vary within the range of from about 0.5 to about 2 mm/day.

The time period $t_c$ is calculated from the date of surgery (not the date of injury). Thus, the time $t_c$ according to Formula I depends on when the device is placed in vivo, i.e. how long time after nerve injury surgery occurs. If for instance surgery occurs on the same day as the injury, $t_c$ is about [14 + (L/v)]. That is, the nerve will be paralysed for about 14 days before the regeneration process starts. However, it shall be noted that even if surgery occurs some days, such as 10 days, after injury, $t_c$ may in some cases still be about [14 + (L/v)] since surgery may affect the lag period.

The approximate time ($t_r$) required for the entire nerve regeneration process to occur using the device according to the invention may be expressed by Formula II:

$$2 \times \left(\frac{L}{v}\right) \leq t_r \leq 14 + 2 \times \left(\frac{L}{v}\right) \qquad (\mathrm{II})$$

The time period $t_r$ is calculated from the date of surgery (not the date of injury). Thus, the time $t_r$ according to Formula II depends on when the device is placed in vivo, i.e. how long time after nerve injury surgery occurs. If for instance surgery occurs on the same day as the injury, $t_r$ is about [14 + 2x(L/v)]. That is, the nerve will generally be paralysed for about 14 days before the nerve regeneration process starts. However, it shall be noted that even if surgery occurs some days, such as 10 days, after injury, $t_r$ may in some cases still be about [14 + 2x(L/v)] since surgery may affect the lag period.

As stated above, the nerve encasement structure should advantageously support the fragile growing nerve even after the regeneration process has established contact between the nerve ends, and said supporting structure should preferably last approximately (about $\pm$ 20%) at least until the entire nerve regeneration process has been completed.

Table 1 shows approximate in vivo degradation times of the guiding means, preferably in the form of fibres, and the encasement structure, respectively, that are believed to be advantageous for varying nerve gap sizes and axon growth rates calculated using formulas (I) and (II), under proviso that surgery occurs on the date of injury.

It shall be noted that the time periods, $t_1$ and $t_2$, given in Table 1 are only approximate and shall only be interpreted as indicative of suitable in vivo degradation times according to the invention.

Table 1

| Gap size [mm] | 0.5 mm/day | | 1 mm/day | | 2 mm/day | |
|---|---|---|---|---|---|---|
| | $t_1$ [days] | $t_2$ [days] | $t_1$ [days] | $t_2$ [days] | $t_1$ [days] | $t_2$ [days] |
| 5 | $24 \pm 5$ | $\geq (34 \pm 7)$ | $19 \pm 4$ | $\geq (24 \pm 5)$ | $17 \pm 3$ | $\geq (19 \pm 4)$ |
| 10 | $34 \pm 7$ | $\geq (54 \pm 11)$ | $24 \pm 5$ | $\geq (34 \pm 7)$ | $19 \pm 4$ | $\geq (24 \pm 5)$ |
| 50 | $114 \pm 23$ | $\geq (214 \pm 43)$ | $64 \pm 13$ | $\geq (114 \pm 23)$ | $39 \pm 8$ | $\geq (64 \pm 13)$ |
| 100 | $214 \pm 43$ | $\geq (414 \pm 83)$ | $114 \pm 23$ | $\geq (214 + 43)$ | $64 \pm 13$ | $\geq (114 \pm 23)$ |
| 200 | $414 \pm 83$ | $\geq (814 \pm 163)$ | $214 \pm 43$ | $\geq (414 \pm 83)$ | $114 \pm 83$ | $\geq (214 \pm 43)$ |

The(second)device according to the invention for promoting regeneration of an injured nerve comprises a biodegradable nerve encasement structure, and a plurality of biodegradable guiding means, preferably a plurality of biodegradable fibres, wherein at least a majority of the guiding means presents an in vivo degradation time $t_1$, at least a major part of the nerve encasement structure presents an in vivo degradation time $t_2$, and $t_2$ being longer than $t_1$ ($t_2 > t_1$).

The in vivo degradation time $t_1$ of said at least a majority of the guiding means of the second device according to the invention is preferably less than or approximately equal to a time $t_c$ required for establishing regenerated contact between the ends of an injured nerve using the device for said regeneration.

The nerve regeneration (first and second) device may be used for healing of both short (defined in rats as essentially no gap to a gap < 10 mm) and long (defined in rats as $\geq$ 10 mm ) nerve gaps. The (first and second) device according to the invention may be used for healing of nerve injuries, such as nerve gaps within the range of from 1 mm to 20 cm or even longer, in mammals, including man.

The (first and second) device is particularly useful for healing of long nerve gaps.

The (first and second) device may be used either for nerve healing in the PNS or in the CNS, including injuries of the spinal cord. Thus, it shall be noted that the term "injured nerve" in connection with the invention includes "injured spinal cord".

The nerve encasement structure is preferably porous to allow passage of substances essential for nerve cell metabolism, such as water, salts, nutrients, etc, and penetration of blood vessels.

The material of the nerve encasement structure and the material of the guiding means should preferably each comprise PHB or more biodegradable polymers. The material of the nerve encasement structure and the material of the guiding means may either comprise the same type of biodegradable polymer(s) or different types of biodegradable polymers.

Examples of biodegradable polymers include biocompatible and biodegradable polyesters; polyorthoesters; polyphosphoesters; polycaprolactam; polyvinyl alcohols; polyanhydrides; polyesteramides; polyamides; polyurethanes; polydioxanes; polyacetals; polyketals; polycarbonates; polyorthocarbonates; polyphosphazens; polyalkylene oxalates; polalkylene succinates; poly(amino acids) (i.e. polypeptides or proteins) ; polyethers, polysaccharides (e.g. alginate); and coplymers, terpolymers or combinations or mixtures thereof.

Biodegradable polyesters include homo- and copolymers of hydroxycarboxylic acids, such as glycolic acids, lactic acids (D-, L- or DL-form), hydroxybutyric acid, hydroxyvaleric acid, trimethyl carbonate, dioxane, and caprolactone.

Examples of biodegradable homo- and copolymers of hydroxycarboxylic acids are polyglycolic acids (also called polyglycolides) (PGA); polylactic acids (also called polylactides) (PLA); polylactic-co-glycolic acids (PLGA); polymalic acids; polyhydroxybutyrate (also called polyhydroxybutyric acid) (PHB); polyhydroxyvalerate (also called polyhydroxyvaleric acid) (PHV); poly(hydroxybutyrate-co-hydroxyvalerate) (PHBV); polytrimethyl carbonates; polydioxanes, such as poly(p-dioxanone) (PDS); and polycaprolactones, such as poly($\varepsilon$-caprolactone) (PCL).

In vivo, the polyesters of hydroxycarboxylic acids undergo random non-enzymatic hydrolysis of backbone ester linkages (bulk degradation) into bioresorbable metabolites. During hydrolysis of the hydrocarboxylic acid polymers, oligomers and/or monomers having carboxylic groups are formed.

The guiding means, preferably a plurality of guiding fibres, in the device according to the invention are made of a material comprising PHB (e.g. poly-3-hydroxybutyrate (P3HB) or poly-4-hydroxybutyrate (P4HB)) or a material comprising polylactic-co-glycolic acids (PLGA).

The nerve encasement structure in the device according to the invention is made of a material comprising PHB (e.g. poly-3-hydroxybutyrate (P3HB) or poly-4-hydroxybutyrate (P4HB)).

Use of PHB in nerve healing has been found to be advantageous since PHB is biocompatible, biodegradable, non-toxic, non-carcinogenic, and non-antigenic and corresponds well to the desired mechanical properties of a nerve healing material, such as strength, flexibility, elasticity, and processability. The biodegradation pattern of PHB also makes the polymer well suited for use in nerve healing.

The in vivo degradation time for a biodegradable polymer, such as PHB or PLGA, may be adjusted by altering the molecular weight of the polymer, e.g. by subjecting the polymer to heat, radiation, a hydrolytic environment, or enzymatic degradation, or by chemical modification of the polymer.

As known to persons skilled in the art, a biodegradable polymer having a lower molecular weight is in vivo degraded faster than the same type of polymer having a higher molecular weight.

Thus, said one or more polymers comprised in the material of the guiding means preferably present(s) an average molecular weight which is lower than an average molecular weight of said one or more polymers comprised in the material of the nerve encasement structure.

The material of the nerve encasement structure in the device (first and second) preferably comprises PHB having an average molecular weight ($M_w$) within the range of from 10 000 to 1 000 000, more preferably from 50 000 to 500 000, and most preferably from 100 000 to 250 000.

The material of the guiding means, preferably fibres, in the device (first and second), comprises PHB having an average molecular weight ($M_w$) within the range of from 10 000 to 1 000 000, more preferably from 50 000 to 500 000, and most preferably from 50 000 to < 250 000, under proviso that the average molecular weight of the guiding means preferably is lower than the molecular weight of the nerve encasement structure in the case where the material of the nerve encasement structure also comprises PHB.

When used in surgical procedures, the nerve encasement structure and the guiding means should exhibit certain mechanical properties, such as strength and flexibility. The structure and the guiding means should also be easy to handle and should preserve their integrity (until a disintegrated state has been reached by degradation and/or dissolution). Moreover, the structure and the guiding means should have a certain biodegradability. Therefore, the PHB material preferably has a molecular weight of at least 50 000. Furthermore, even though no negative effect is observed when a porous nerve encasement structure encompasses a regenerated nerve, there is no need for the device to last longer than necessary in the body of the patient. Thus, the molecular weight of the PHB material is preferably equal to or below 1 000 000, more preferably equal to or below 500 000.

The material of the guiding means, preferably fibres, in the device (first and second) , comprises PLGA having an average molecular weight ($M_w$) within the range of from 20 000 to 200 000.

As known to persons skilled in the art, the in vivo degradation time of PLGA is affected by the molar ratio of glycolide and lactide (D-, L- or DL-form) in the copolymer. When guiding means of PLGA are used in the device according to the invention, the PLGA should preferably comprise 50-90% glycolide and 10-50% lactide.

It shall also be noted that in some cases, physicians might consider it advantageous that the device is in an essentially disintegrated state when the nerve regeneration process has been finalized, i.e. at approximate ($\pm$ 20%) $t_r$ days after surgery. As a consequence, the molecular weight of the biodegradable polymer of the nerve encasement structure may be selected in accordance with the gap size of the injured nerve. Since a shorter nerve gap is bridged and regenerated faster than a longer gap, a polymer having a lower molecular weight and thus a shorter degradation time may be selected for the healing of a shorter nerve gap in comparison to the healing of a longer nerve gap.

A preferred embodiment of the (first and second) device includes a nerve encasement structure comprising a compressed non-woven sheet of biodegradable PHB fibres, preferably PHB fibres, having an essentially unidirectional fibre orientation. Said sheet is preferably formed into a tubular structure during in vivo application thereof (the sheet is wrapped around the nerve ends) and said fibre orientation is then oriented along the longitudinal axis of the tubular structure. The sheet is in vivo preferably kept in the form of a tubular structure by use of for instance a glue, such as a fibrin glue, sutures through the sheet, or frictional engagement.

Preferably, the non-woven sheet, forming a nerve encasement structure in vivo, has a thickness within the range of 0.1-0.4 mm, more preferably 0.2-0.3 mm. Moreover, the non-woven sheet preferably has a weight per unit area within the range of 9-11 mg/cm$^2$.

The plurality of guiding means, preferably PHB fibres, or PHB fibres and PLGA fibres, may be in the form of individual guiding means, such as individual fibres (monofilaments) and/or a guiding means matrix, such as a fibre matrix.

A preferred embodiment of the (first and second) device includes a plurality of biodegradable PHB fibres and PLGA fibres, in the form of non-woven, preferably non-compressed (i.e. a non-bonded fibre web), having an essentially unidirectional fibre orientation, which in use is oriented along the direction of desired nerve growth.

Furthermore, since the amount of the guiding means, in the lumen of the nerve tube has been found to affect the amount of regenerated axons obtained, the guiding means, advantageously in the form of fibres, should preferably occupy $\leq$ 2.0% by volume of the lumen formed by the nerve encasement structure of the device according to the invention.

It is believed that the guiding means preferably should occupy at least 0.05% by volume of said lumen. The guiding means may, for instance, occupy $\leq$ 1.0% by volume of said lumen, such as within the range of from 0.2 to 1.0%.

It is also believed that not only the amount but also the size of the guiding means, such as guiding fibres, in the lumen of the nerve tube affects the amount of regenerated axons obtained.

The inventors have found that each guiding means, which advantageously is in the form of a fibre, preferably should have a cross-sectional dimension similar to the cross-sectional dimension of the axons (about 2-20 $\mu$m). Also the cross-sectional dimension of the bands of Büngner is of similar dimension. Thus, preferably each fibre of a majority of the guiding means, advantageously in the form of fibres, in the device according to the invention should have a cross-sectional dimension $\leq$ 50 $\mu$m, more preferably $\leq$ 20 $\mu$m, such as within the range of from 1 to 20 $\mu$m and in particular within the range of from 5 to 15 $\mu$m.

It shall also be noted that it might be advantageous to coat the guiding means, such as guiding fibres, with so-called extracellular matrix molecules, such as laminin, fibronectin and/or collagen.

The (first and second) device may further comprise a hydrogel, preferably within the lumen of the nerve encasement structure. The guiding means, preferably fibres, may then be dispersed, preferably homogeneously, in the hydrogel matrix.

Examples of hydrogel materials include agarose; alginate; chitosan; collagen; laminin; cross-linked polyethylene oxide; cross-linked hyaluronic acid; and polyvinylalcohol.

It shall be noted that the hydrogel material may be in a dehydrated state when applied in vivo. The material is in that case hydrated in vivo by body fluids thus forming a hydrogel.

Alternatively, the dehydrated hydrogel material may be hydrated in situ, by for instance the physician performing the surgery, prior to application in vivo.

It may also be advantageous to comprise one or more biologically active substances or cells, such as a nerve growth promoting substance selected from the group consisting of nerve growth factor (NGF); brain-derived neurotrophic factor (BDNF); neurotrophin-3 (NT-3); neurotrophin-4 (NT-4); glial growth factor (GGF); insulin-like growth factor (IGF);

platelet-derived growth factor (PDGF); fibroblast growth factor (FGF); transforming growth factor (TGF); epidermal growth factor (EGF); endothelial cells; fibroblasts; Schwann cells; olfactory ensheathing cells (a type of glial cells), stem cells or precursor cells thereof, in the (first and second) device according to the invention.

Reference is made to a first kit for preparing the above described first device for promoting regeneration of an injured nerve. Said first kit comprises a sheet, preferably biodegradable, and a plurality of biodegradable guiding means, wherein at least a majority of the guiding means presents an in vivo degradation time $t_1$ being less than or approximately equal to a time $t_c$ required for establishing regenerated contact between the ends of an injured nerve using the device for said regeneration.

The invention also relates to a second kit for preparing the above described second device according to the invention for promoting regeneration of an injured nerve. Said second kit comprises a biodegradable sheet and a plurality of biodegradable guiding means, wherein at least a majority of the guiding means presents an in vivo degradation times $t_1$, at least a major part of the sheet presents an in vivo degradation time $t_2$, and $t_2$ being longer than $t_1$ ($t_2 > t_1$).

The sheet is when used surgically formed into a nerve encasement structure, preferably in the form of a tubular structure, such as a conduit.

It shall be noted that the (first and second) kit may comprise a sheet already formed into a tubular structure (sheet in tubular form).

The considerations and preferred embodiments of the (first and second) kit are analogous to the considerations and preferred embodiments described above in relation to the (first and second) device.

As described above, it may be advantageous to further comprise a hydrogel matrix within the lumen of the nerve encasement structure. Thus, the (first and second) kit may comprise a hydrogel material, preferably a hydrogel material in a dehydrated state.

A hydrogel may be applied onto the sheet, and then dehydrated. Thus, the sheet comprised in the (first and second) kit may comprise a coating of a dehydrated hydrogel material.

The (first and second) kit may further comprise distilled water for hydration of the dehydrated hydrogel material.

Furthermore, the (first and second) kit may also comprise one or more biologically active substances or cells, such as a nerve growth promoting substance selected from the group consisting of nerve growth factor (NGF); brain-derived neurotrophic factor (BDNF); neurotrophin-3 (NT-3); neurotrophin-4 (NT-4); glial growth factor (GGF); insulin-like growth factor (IGF); platelet-derived growth factor (PDGF); fibroblast growth factor (FGF); transforming growth factor (TGF); epidermal growth factor (EGF); endothelial cells; fibroblasts; Schwann cells; olfactory ensheathing cells; stem cells or precursor cells thereof.

An example of a method of manufacturing the (first or second) device using the (first or second) kit according to the invention comprises:

- dispersing (in vitro) the plurality of biodegradable guiding means, preferably a plurality of fibres, in a hydrogel (before or after hydration thereof),
- applying the hydrogel and the guiding means on at least a part of at least one surface of the sheet (in this embodiment, the hydrogel comprises dispersed guiding means),
- dehydrating the hydrogel-coated sheet, and
- forming (preferably in vivo) the coated sheet into a nerve encasement structure, preferably a tubular structure, wherein said coating faces the lumen of the nerve encasement structure.

In this case, a kit comprising a sheet, biodegradable guiding means, preferably in the form of fibres, and a dehydrated hydrogel material is integrally formed as an implantable body.

Thus, reference is made to a first biodegradable sheet for preparing the first device according to the invention for promoting regeneration of an injured nerve, said sheet having at least one surface at least partly coated with a dehydrated hydrogel material and a plurality of biodegradable guiding means, preferably in the form of fibres, wherein at least a majority of the guiding means presents an in vivo degradation time $t_1$ being less than or approximately equal to a time $t_c$ required for establishing regenerated contact between the ends of an injured nerve using device.

Further, reference is made to a second biodegradable sheet for preparing the second device according to the invention for promoting regeneration of an injured nerve, said sheet having at least one surface at least partly coated with a dehydrated hydrogel material and a plurality of biodegradable guiding means, preferably in the form of fibres, wherein at least a majority of the guiding means presents an in vivo degradation time $t_1$, at least a major part of the sheet presents an in vivo degradation time $t_2$, and $t_2$ being longer than $t_1$ ($t_2 > t_1$).

Said dehydrated hydrogel material of the above disclosed (first and second) biodegradable sheet according to the invention may also advantageously comprise one or more of the above mentioned biologically active substances or cells. The (first or second) kit or the (first or second) biodegradable sheet may be used to produce the (first or second).

The (first or second) kit or the (first or second) biodegradable sheet may also be used in a method for repairing an injured nerve. Such a method comprises at least partly encasing the ends of the injured nerve and the plurality of guiding means, preferably fibres, using the sheet preferably formed into a tubular structure, such as a conduit.

Thus, a method for promoting regeneration of an injured nerve may comprise the step of applying at said injured nerve the (first or second) device.

Also, biodegradable guiding means, preferably in the form of fibres, may be used for promoting regeneration of an injured nerve, wherein at least a majority of the guiding means presents an in vivo degradation time $t_1$ being less than or approximately equal to a time $t_c$ required for establishing regenerated contact between the ends of an injured nerve using the guiding means for said regeneration.

The guiding means are preferably made from a material comprising one or more biodegradable polymers, more preferably a biodegradable polyester, such as PHB or PLGA.

The average molecular weight of said PHB is preferably within the range of from 50 000 to 250 000.

The average molecular weight of said PLGA is preferably within the range of from 20 000 to 200 000. Moreover, said PLGA preferably comprises 50-90 % glycolide and 10-50 % lactide.

The plurality of guiding means may be in the form of individual guiding means, such as individual fibres, and/or a guiding means matrix, such as a fibre matrix.

The guiding means are preferably fibres in the form of a fibre matrix, such as a non-compressed non-woven (i.e. a non-bonded fibre web) having an essentially unidirectional fibre orientation.

Example

Nerve conduits are constructed from a nonwoven sheet of polyhydroxybutyrate (PHB) having an average molecular weight of 140 000, a sheet thickness of 0.25 mm and a weight per unit area of 10 mg/cm$^2$. The conduits are evaluated for their ability to cross a 1 cm gap in the sciatic nerve of rats.

The conduits are filled up to a certain percentage of volume, 0.2%, 0.5% or 0.9%, with a non-bonded PHB fibre web having an essentially unidirectional fibre direction. The density of PHB is 1.24 g/cm$^3$. 0.35 mg non-bonded PHB fibre web occupies about 0.9% by volume of the conduit lumen.

The PHB fibres of said non-bonded fibre web have an average molecular weight of 80 000 and cross-sectional dimensions within the range of 5-15 $\mu$m.

A segment of the sciatic nerve in rats is excised to leave a 10 mm gap in the nerve. The PHB conduit is inserted to bridge the gap and secured to the nerve ends with two 9/0 nylon sutures (supplied by Ethicon) at each end.

Following grafting, the conduit is injected with a Schwann cell suspension.

The intraluminar fibres act both as a matrix for the seeded Schwann cells providing a homogeneous distribution thereof and as a guide to stimulate unidirectional axon regeneration.

While the invention has been described in detail and with reference to specific embodiments thereof, it will be apparent for one skilled in the art that various changes and modifications can be made therein without departing from the scope thereof.

**Claims**

1. A device (1) for promoting regeneration of an injured nerve comprising a biodegradable nerve encasement structure (2), and a plurality of biodegradable guiding means (3) wherein the material of the nerve encasement structure and the material of the guiding means each comprises PHB or PHB and more biodegradable polymers, **characterized in that** said PHB or PHB and more polymers comprised in the material of the guiding means present an average molecular weight which is lower than an average molecular weight of said PHB or PHB and more polymers comprised in the material of the nerve encasement structure, and the PHB average molecular weight of the nerve encasement structure being within the range of from 100 000 to 250 000 and the PHB average molecular weight of the guiding means being within the range of from 50 000 to < 250 000, so that at least a majority of the guiding means presents an in vivo degradation time $t_1$, at least a major part of the nerve encasement structure presents an in vivo degradation time $t_2$, and $t_2$ being longer than $t_1$ ($t_2 > t_1$).

2. A device according to claim 1, wherein the plurality of biodegradable guiding means are a plurality of biodegradable guiding fibres.

3. A device according to any one of the preceding claims, wherein the nerve encasement structure comprises a compressed non-woven sheet of biodegradable fibres having an essentially unidirectional fibre orientation.

4. A device according to any one of the preceding claims, wherein the plurality of guiding means are biodegradable fibres in the form of a non-bonded fibre web having an essentially unidirectional fibre orientation.

**5.** A device according to any one of the preceding claims, further comprising a hydrogel matrix.

**6.** A device according to any one of the preceding claims, further comprising one or more biologically active substances or cells.

**7.** A device according to claim 6, wherein said one or more biologically active substances comprises a nerve growth promoting substance selected from the group consisting nerve growth factor (NGF); brain-derived neurotrophic factor (BDNF) ; neurotrophin-3 (NT-3); neurotrophin-4 (NT-4); glial growth factor (GGF); insulin-like growth factor (IGF); platelet-derived growth factor (PDGF); fibroblast growth factor (FGF); transforming growth factor (TGF); and epidermal growth factor (EGF).

**8.** A device according to claim 6, wherein said one or more biologically active cells is selected from the group consisting of endothelial cells; fibro-blasts; Schwann cells; olfactory ensheathing cells; stem cells or precursor cells thereof.

**9.** A device according to any one of the preceding claims, wherein the guiding means occupies < 2.0 % by volume of the lumen formed by the nerve encasement structure.

**10.** A device according to any one of the preceding claims, wherein each guiding means of a majority of the guiding means has a cross-sectional dimension $\leq 50\ \mu$m.

**11.** A device according to claim 10, wherein each guiding means of a majority of the guiding means has a cross-sectional dimension $\leq 20\ \mu$m.

**12.** A device according to claim 11, wherein each guiding means of a majority of the guiding means has a cross-sectional dimension within the range of from 5 to 15 $\mu$m.

**13.** A kit for preparing a device for promoting regeneration of an injured nerve, said kit comprising a biodegradable sheet (2) and a plurality of biodegradable guiding means (3), wherein the material of the sheet and the material of the guiding means each comprises PHB or PHB and more biodegradable polymers, **characterized in that** said PHB or PHB and more polymers comprised in the material of the guiding means present an average molecular weight which is lower than an average molecular weight of said PHB or more polymers comprised in the material of the sheet, the material of the sheet and the material of the guiding means each comprising PHB and the PHB molecular weight of the sheet being within the range of from 100 000 to 250 000 and the PHB molecular weight of the guiding means being within the range of from 50 000 to < 250 000, so that at least a majority of the guiding means presents an in vivo degradation times $t_1$, at least a major part of the sheet presents an in vivo degradation time $t_2$, and $t_2$ being longer than $t_1$, ($t_2 > t_1$).

**14.** A kit according to claim 13, wherein the plurality of biodegradable guiding means are a plurality of biodegradable guiding fibres.

**15.** A kit according to claim 13 or 14, wherein the sheet comprises a compressed non-woven sheet of biodegradable fibres having an essentially unidirectional fibre orientation.

**16.** A kit according to any one of claims 13-15, wherein the plurality of guiding means are biodegradable fibres in the form of a non-bonded fibre web having an essentially unidirectional fibre orientation.

**17.** A kit according to any one of claims 13-16, further comprising a hydrogel material.

**18.** A kit according to claim 17, wherein the hydrogel is in a dehydrated state.

**19.** A kit according to any one of claims 13-18, further comprising one or more biologically active substances or cells.

**20.** A kit according to claim 19, wherein said one or more biologically active substance comprises a nerve growth promoting substance selected from the group consisting of nerve growth factor (NGF); brain-derived neurotrophic factor (BDNF); neurotrophin-3 (NT-3); neurotrophin-4 (NT-4); glial growth factor (GGF); insulin-like growth factor (IGF); platelet-derived growth factor (PDGF); fibroblast growth factor (FGF); transforming growth factor (TGF); and epidermal growth factor (EGF).

**21.** A kit according to claim 19, wherein said one or more biologically active cells is selected from the group consisting of endothelial cells; fibroblasts; Schwann cells; olfactory ensheathing cells; stem cells or precursor cells thereof.

**Patentansprüche**

**1.** Vorrichtung (1) zur Förderung der Regeneration eines verletzten Nervs, die eine biologisch abbaubare, den Nerv umhüllende Führungsstruktur (2) und mehrere biologisch abbaubare Führungsmittel (3) umfasst, wobei das Material von der den Nerv umhüllenden Führungsstruktur und das Material von den Führungsmitteln jeweils PHB oder PHB und weitere biologisch abbaubare Polymere umfasst, **dadurch gekennzeichnet, dass** das PHB oder das PHB und weitere Polymere, die in dem Material von den Führungsmitteln enthalten sind, ein durchschnittliches Molekulargewicht vorweisen, dass niedriger ist als ein durchschnittliches Molekulargewicht von dem PHB oder dem PHB und weiteren Polymeren, die in dem Material von der den Nerv umhüllenden Führungsstruktur enthalten sind und das durchschnittliche Molekulargewicht des PHB von der den Nerv umhüllenden Führungsstruktur innerhalb des Bereiches von 100.000 bis 250.000 liegt und das durchschnittliche Molekulargewicht des PHB von den Führungsmitteln innerhalb des Bereiches von 50.000 bis <250.000 derart liegt, dass mindestens ein überwiegender Anteil der Führungsmittel in vivo eine Abbauzeit $t_1$ vorweist, mindestens ein überwiegender Anteil von der den Nerv umhüllenden Führungsstruktur in vivo eine Abbauzeit $t_2$ vorweist, und wobei $t_2$ länger als $t_1$ ist ($t_2 > t_1$).

**2.** Vorrichtung nach Anspruch 1, wobei die mehrere biologisch abbaubaren Führungsmittel mehrere biologisch abbaubare Führungsfasern sind.

**3.** Vorrichtung nach einem der vorhergehenden Ansprüche, wobei die den Nerv umhüllende Führungsstruktur ein verdichtetes Faservlies aus biologisch abbaubaren Fasern umfasst, die im Wesentlichen eine in eine Richtung verlaufende Faserausrichtung aufweisen.

**4.** Vorrichtung nach einem der vorhergehenden Ansprüche, wobei die mehrere Führungsmittel biologisch abbaubare Fasern in der Form eines nicht verbundenen Fasergewebes sind, die im Wesentlichen eine in eine Richtung verlaufende Faserausrichtung aufweisen.

**5.** Vorrichtung nach einem der vorhergehenden Ansprüche, die ferner eine Hydrogel-Matrix umfasst.

**6.** Vorrichtung nach einem der vorhergehenden Ansprüche, die ferner eine oder mehrere biologisch aktive Substanzen oder Zellen umfasst.

**7.** Vorrichtung nach Anspruch 6, wobei die eine oder mehrere biologisch aktive Substanzen eine das Nervenwachstum fördernde Substanz umfassen, die ausgewählt ist aus der Gruppe, die aus Nervenwachstumsfaktor (NGF), Brain-derived Neurotrophic Factor (BDNF); Neurotrophin-3 (NT-3); Neurotrophin-4 (NT-4); glialer Wachstumsfaktor (GGF); insulinähnlicher Wachstumsfaktor (IGF); Platelet-derived Growth Factor (PDGF); Fibroblasten-Wachstumsfaktor (FGF); Transforming Growth Factor (TGF) und Epidermalem Wachstumsfaktor (EGF) besteht.

**8.** Vorrichtung nach Anspruch 6, wobei die eine biologisch aktive oder mehrere biologisch aktive Zellen ausgewählt ist/sind aus der Gruppe, die aus Endothelzellen, Fibroblasten, Schwann-Zellen, olfaktorischen Hüllzellen (olfactory ensheathing cells), Stammzellen oder Vorläuferzellen davon besteht.

**9.** Vorrichtung nach einem der vorhergehenden Ansprüche, wobei die Führungsmittel <2,0 Volumenprozent von dem Lumen in Anspruch nehmen, das durch die den Nerv umhüllende Führungsstruktur gebildet ist.

**10.** Vorrichtung nach einem der vorhergehenden Ansprüche, wobei jedes Führungsmittel von einem überwiegenden Anteil der Führungsmittel eine Querschnittsabmessung von <50 $\mu$m aufweist.

**11.** Vorrichtung nach Anspruch 10, wobei jedes Führungsmittel von einem überwiegenden Anteil der Führungsmittel eine Querschnittsabmessung von <20 $\mu$m aufweist.

**12.** Vorrichtung nach Anspruch 11, wobei jedes Führungsmittel von einem überwiegenden Anteil der Führungsmittel eine Querschnittsabmessung innerhalb des Bereiches von 5 bis 15 $\mu$m aufweist.

**13.** Kit zur Herstellung einer Vorrichtung zur Förderung der Regeneration eines verletzten Nervs, wobei das Kit ein biologisch abbaubares Faservlies (2) und mehrere biologisch abbaubare Führungsmittel (3) umfasst, wobei das Material von dem Faservlies und das Material von den Führungsmitteln jeweils PHB oder PHB und weitere biologisch abbaubare Polymere umfasst, **dadurch gekennzeichnet, dass** das PHB oder das PHB und weitere Polymere, die in dem Material von den Führungsmitteln enthalten sind, ein durchschnittliches Molekulargewicht vorweisen, dass niedriger ist als ein durchschnittliches Molekulargewicht von dem PHB oder dem PHB und weiteren Polymeren, die in dem Material von dem Faservlies enthalten sind, wobei das Material von dem Faservlies und das Material von den Führungsmitteln jeweils PHB enthalten und das Molekulargewicht des PHB von dem Faservlies innerhalb des Bereiches von 100.000 bis 250.000 liegt und das Molekulargewicht des PHB von den Führungsmitteln innerhalb des Bereiches von 50.000 bis <250.000 derart liegt, dass mindestens ein überwiegender Anteil der Führungsmittel in vivo eine Abbauzeit $t_1$ vorweist, mindestens ein überwiegender Anteil von dem Faservlies in vivo eine Abbauzeit $t_2$ vorweist, und wobei $t_2$ länger als $t_1$ ist ($t_2 > t_1$).

14. Kit nach Anspruch 13, wobei der überwiegende Anteil der biologisch abbaubaren Führungsmittel mehrere biologisch abbaubare Führungsfasern sind.

15. Kit nach Anspruch 13 oder 14, wobei das Faservlies ein verdichtetes Faservlies aus biologisch abbaubaren Fasern umfasst, die im Wesentlichen eine in einer Richtung verlaufende Faserausrichtung aufweisen.

16. Kit nach einem der Ansprüche 13 - 15, wobei die mehrere Führungsmittel biologisch abbaubare Fasern in der Form eines nicht verbundenen Fasergewebes sind, die im Wesentlichen eine in eine Richtung verlaufende Faserausrichtung aufweisen.

17. Kit nach einem der Ansprüche 13-16, das ferner ein Hydrogel-Material umfasst.

18. Kit nach Anspruch 17, wobei das Hydrogel in einem entwässerten Zustand ist.

19. Kit nach einem der vorhergehenden Ansprüche 13-18, das ferner eine oder mehrere biologisch aktive Substanzen oder Zellen umfasst.

20. Kit nach Anspruch 19, wobei die eine oder mehrere biologisch aktive Substanzen eine das Nervenwachstum fördernde Substanz umfasst, die ausgewählt ist aus der Gruppe, die aus Nervenwachstumsfaktor (NGF), Brain-derived Neurotrophic Factor (BDNF); Neurotrophin-3 (NT-3); Neurotrophin-4 (NT-4); glialer Wachstumsfaktor (GGF); insulinähnlicher Wachstumsfaktor (IGF); Platelet-derived Growth Factor (PDGF); Fibroblasten-Wachstumsfaktor (FGF); Transforming Growth Factor (TGF) und Epidermaler Wachstumsfaktor (EGF) besteht.

21. Kit nach Anspruch 19, wobei die eine oder mehrere biologisch aktive Zellen ausgewählt ist/sind aus der Gruppe, die aus Endothelzellen, Fibroblasten, Schwann-Zellen, olfaktorischen Hüllzellen (olfactory ensheathing cells), Stammzellen oder Vorläuferzellen davon besteht.

**Revendications**

1. Dispositif (1) destiné à favoriser la régénération d'un nerf lésé, qui comprend une structure de gaine biodégradable pour nerfs (2) et une pluralité de moyens de guidage biodégradables (3), le matériau de la structure de gaine pour nerfs et le matériau des moyens de guidage comprenant chacun des polymères biodégradables de PHB ou de PHB et autres, **caractérisé en ce que** lesdits polymères de PHB ou de PHB et autres compris dans le matériau des moyens de guidage présentent une masse moléculaire moyenne qui est inférieure à une masse moléculaire moyenne desdits polymères de PHB ou PHB et autres compris dans le matériau de la structure de gaine pour nerfs, et la masse moléculaire moyenne du PHB de la structure de gaine pour nerfs se trouvant dans la plage de 100 000 à 250 000 et la masse moléculaire moyenne du PHB des moyens de guidage se trouvant dans la plage de 50 000 à moins de 250 000, de manière à ce qu'au moins une majorité des moyens de guidage présente un temps de dégradation in vivo $t_1$, au moins une majorité de la structure de gaine pour nerfs présente un temps de dégradation in vivo $t_2$, et $t_2$ étant plus long que $t_1$ ($t_2 > t_1$).

2. Dispositif selon la revendication 1, dans lequel la pluralité de moyens de guidage biodégradables sont une pluralité de fibres de guidage biodégradables.

3. Dispositif selon l'une quelconque des revendications précédentes, dans lequel la structure de gaine pour nerfs comprend une feuille non tissée compressée de fibres biodégradables ayant une orientation des fibres sensiblement unidirectionnelle.

4. Dispositif selon l'une quelconque des revendications précédentes, dans lequel la pluralité de moyens de guidage sont des fibres biodégradables sous la forme d'une nappe fibreuse non liée ayant une orientation des fibres sensiblement unidirectionnelle.

5. Dispositif selon l'une quelconque des revendications précédentes, qui comprend en outre une matrice d'hydrogel.

6. Dispositif selon l'une quelconque des revendications précédentes, qui comprend en outre une ou plusieurs substances ou cellules biologiquement actives.

7. Dispositif selon la revendication 6, dans lequel ladite ou lesdites substances biologiquement actives comprennent une substance favorisant la croissance des nerfs choisie dans le groupe constitué par le facteur de croissance des nerfs (NGF) ; le facteur neurotrophique dérivé du cerveau (BDNF); la neurotrophine 3 (NT-3) ; la neurotrophine 4 (NT-4) ; le facteur de croissance gliale (GGF) ; le facteur de croissance de type insuline (IGF) ; le facteur de croissance dérivé des plaquettes (PDGF) ; le facteur de croissance des fibroblastes (FGF) ; le facteur de croissance transformant (TGF) ; et le facteur de croissance épidermique (EFG).

8. Dispositif selon la revendication 6, dans lequel ladite ou lesdites cellules biologiquement actives sont choisies dans le groupe constitué par les cellules endothéliales ; les fibroblastes ; les cellules de Schwann ; les cellules olfactives engainantes ; les cellules souches ou cellules précurseurs de celles-ci.

9. Dispositif selon l'une quelconque des revendications précédentes, dans lequel les moyens de guidage occupent moins de 2,0 % en volume de la lumen formée par la structure de gaine pour nerfs.

10. Dispositif selon l'une quelconque des revendications précédentes, dans lequel chacun des moyens de guidage d'une majorité des moyens de guidage a une dimension transversale inférieure ou égale à 50 $\mu$m.

11. Dispositif selon la revendication 10, dans lequel chacun des moyens de guidage d'une majorité des moyens de guidage a une dimension transversale inférieure ou égale à 20 $\mu$m.

12. Dispositif selon la revendication 11, dans lequel chacun des moyens de guidage d'une majorité des moyens de guidage a une dimension transversale comprise dans la plage de 5 à 15 $\mu$m.

13. Kit de préparation d'un dispositif destiné à favoriser la régénération d'un nerf lésé, ledit kit comprenant une feuille biodégradable (2) et une pluralité de moyens de guidage biodégradables (3), le matériau de la feuille et le matériau des moyens de guidage comprenant chacun des polymères biodégradables de PHB ou de PHB et autres, **caractérisé en ce que** lesdits polymères de PHB ou de PHB et autres compris dans le matériau des moyens de guidage présentent une masse moléculaire moyenne qui est inférieure à une masse moléculaire moyenne desdits polymères de PHB ou PHB et autres compris dans le matériau de la feuille, le matériau de la feuille et le matériau des moyens de guidage comprenant chacun du PHB et la masse moléculaire du PHB de la feuille se trouvant dans la plage de 100 000 à 250 000 et la masse moléculaire du PHB des moyens de guidage se trouvant dans la plage de 50 000 à moins de 250 000, de manière à ce qu'au moins une majorité des moyens de guidage présente un temps de dégradation in vivo $t_1$, au moins une majorité de la feuille présente un temps de dégradation in vivo $t_2$, et $t_2$ étant plus long que $t_1$ ($t_2 > t_1$).

14. Kit selon la revendication 13, dans lequel la pluralité de moyens de guidage biodégradables sont une pluralité de fibres de guidage biodégradables.

15. Kit selon la revendication 13 ou 14, dans lequel la feuille comprend une feuille non tissée compressée de fibres biodégradables ayant une orientation des fibres sensiblement unidirectionnelle.

16. Kit selon l'une quelconque des revendications 13 à 15, dans lequel la pluralité de moyens de guidage sont des fibres biodégradables sous la forme d'une nappe fibreuse non liée ayant une orientation des fibres sensiblement unidirectionnelle.

17. Kit selon l'une quelconque des revendications 13 à 16, qui comprend en outre un matériau d'hydrogel.

**18.** Kit selon la revendication 17, dans lequel l'hydrogel se trouve dans un état déshydraté.

**19.** Kit selon l'une quelconque des revendications 13 à 18, qui comprend en outre une ou plusieurs substances ou cellules biologiquement actives.

**20.** Kit selon la revendication 19, dans lequel ladite ou lesdites substances biologiquement actives comprennent une substance favorisant la croissance des nerfs choisie dans le groupe constitué par le facteur de croissance des nerfs (NGF) ; le facteur neurotrophique dérivé du cerveau (BDNF) ; la neurotrophine 3 (NT-3) ; la neurotrophine 4 (NT-4) ; le facteur de croissance gliale (GGF) ; le facteur de croissance de type insuline (IGF) ; le facteur de croissance dérivé des plaquettes (PDGF) ; le facteur de croissance des fibroblastes (FGF) ; le facteur de croissance transformant (TGF); et le facteur de croissance épidermique (EFG).

**21.** Kit selon la revendication 19, dans lequel ladite ou lesdites cellules biologiquement actives sont choisies dans le groupe constitué par les cellules endothéliales ; les fibroblastes ; les cellules de Schwann ; les cellules olfactives engainantes ; les cellules souches ou cellules précurseurs de celles-ci.

*Fig. 1*

**EP 1 643 935 B1**

**REFERENCES CITED IN THE DESCRIPTION**

**Patent documents cited in the description**

- WO 9737002 A
- WO 0181552 A
- WO 8806871 A

- EP 1201256 A
- EP 0945145 A
- US 6548569 B